# EUROPÄISCHE PATENTANMELDUNG

(11) **EP 2 698 424 A1**
(43) Veröffentlichungstag der Anmeldung: **19.02.2014**
(21) Anmeldenummer: 13179821.7
(22) Anmeldetag: 09.08.2013
(51) Int. Cl.: C12M 1/107, C12M 1/00, C12M 1/12

(54) **Verfahren zum Anfahren einer Biogasanlage**

(30) Priorität: 13.08.2012 DE 102012107396
(71) Anmelder: 4Biogas GmbH & Co. KG, 44229 Dortmund (DE)
(72) Erfinder: Brachthäuser, Dr., Benno, 44229 Dortmund (DE); Kiomall, Daniel, 44137 Dortmund (DE); Bexten, Matthias, 33378 Rheda-Wiedenbrück (DE); Karl, Rainer, 99718 Clingen (DE)
(74) Vertreter: Meinke, Dabringhaus und Partner GbR

(57) **Zusammenfassung**

Die Erfindung betrifft ein Verfahren zum Anfahren von Biogasanlagen (1), bei dem ein Animpfkonzentrat (28) erzeugt wird. Um das Verfahren derart weiterzuentwickeln, dass die für den Anfahrprozess bedeutende Erzeugung des Animpfkonzentrates (28) weiter vereinfacht und optimiert wird, sieht die Erfindung folgende Verfahrensschritte vor:
a) Erzeugung eines Filtratstromes (11) aus einem Filtrat;
b) Zuführung des Filtratstromes (11) in einen mit Füllkörpern (26) beschickten Reaktor (22);
c) Erzeugung des Animpfkonzentrates (28) in dem Reaktor (22);
d) Entnahme des Animpfkonzentrates (28) aus dem Reaktor (22);
e) Abführung des Filtratstromes (11) aus dem Reaktor (22).

## Beschreibung

Die Erfindung betrifft ein Verfahren gemäß dem Oberbegriff des Anspruches 1. Zudem betrifft die Erfindung eine Biogasanlage.

Das Anfahren von Biogasanlagen ist ein zeitaufwändiger Prozess. Bevor die Befüllung des Fermenters erhöht werden kann, muss sichergestellt werden, dass sich eine stabile Biologie in dem Fermenter aufgebaut hat. Ist dies nicht der Fall, kann ein zweistufiges System schnell zusammenbrechen, da die Produkte der ersten Abbaustufe ab einer gewissen Konzentration die zweite Abbaustufe hemmen können.

Wird eine Biogasanlage thermophil betrieben, d.h. bei einer Temperatur zwischen 51 und 55°C, so muss die Anlage zunächst mesophil, d.h. bei einer Temperatur von 30 bis 41°C angefahren werden und dann sehr vorsichtig nach und nach auf die gewünschte thermophile Zieltemperatur gebracht werden. Da insbesondere die thermophilen Mikroorganismen für den Biogasprozess extrem temperaturempfindlich sind, kann dieser Vorgang sich oftmals über einen Zeitraum von 3 bis 6 Monaten erstrecken. In dieser Zeit fehlt allerdings der Energieertrag zur Amortisation der Anlage, so dass eine Beschleunigung des Anfahrprozesses von enormem wirtschaftlichem Interesse ist.

Im Stand der Technik ist man daher dazu übergegangen, große Mengen an Gärsubstrat aus einer vorhandenen Biogasanlage in die anzufahrende Biogasanlage zu pumpen. Diese gängige Praxis ist aber aufgrund des hohen Materialvolumens relativ kostenintensiv und auch nur für mesophile (30 bis 41°C) Biogasanlagen interessant, da es einerseits nur sehr wenige thermophil betriebene Anlagen gibt und das Substrat aus einer thermophilen Anlage andererseits durch den Temperaturabfall beim Transport schnell an Aktivität verliert.

Ein aus dem Stand der Technik bekannter vielversprechender Ansatz, den Anfahrprozess insbesondere einer thermophilen Biogasanlage zu beschleunigen, ist die Erzeugung eines hochkonzentrierten Animpfkonzentrates, welches eine so hohe Aktivität besitzt, dass bereits geringe Mengen ausreichen, um ein großes Volumen ausreichend mit den benötigten Mikroorganismen zu versorgen.

Es ist deshalb Aufgabe der vorliegenden Erfindung, ein Verfahren der eingangs genannten Art derart weiterzuentwickeln, die für den Anfahrprozess bedeutende Erzeugung des hochkonzentrierten Animpfkonzentrates weiter zu vereinfachen und zu optimieren.

Diese Aufgabe wird mit den Merkmalen des Anspruches 1 gelöst. Vorteilhafte Ausgestaltungen der Erfindung ergeben sich aus den Unteransprüchen.

Die Erfindung sieht folgende Verfahrensschritte vor:
a) Erzeugung eines Filtratstromes aus einem Filtrat;
b) Zuführung des Filtratstromes in einen mit Füllkörpern beschickten Reaktor;
c) Erzeugung des Animpfkonzentrates in dem Reaktor;
d) Entnahme des Animpfkonzentrates aus dem Reaktor;
e) Abführung des Filtratstromes aus dem Reaktor.

Kernidee der Erfindung ist es, einen Filtratstrom, der beispielsweise bei der Trennung eines wässrigen Substrates in ein Konzentrat und Filtrat in einer Biogasanlage erzeugt wird, für die Erzeugung eines hochkonzentrierten Animpfkonzentrates weiter zu verwenden.

Der Vorteil der Erfindung ist es, unter Schonung von vorhandenen Ressourcen, in diesem Fall also die Verwendung eines in einer Biogasanlage sowieso schon vorhandenen Filtratstromes, ein hoch konzentriertes Animpfkonzentrat zu erzeugen.

Hierzu wird im Rahmen von weiteren Verfahrensschritten der Filtratstrom zunächst in einen mit Füllkörpern beschickten Reaktor zugeführt, um in dem Reaktor das Animpfkonzentrat zu erzeugen. Der Filtratstrom wird aus dem Reaktor weiter in ein Substratlager befördert - das Animpfkonzentrat reichert sich im unteren Bereich des Reaktors an und kann bei Bedarf zum Animpfen einer Biogasanlage genutzt werden.

Um eine Verstopfung bei der Erzeugung des Animpfkonzentrates in dem Reaktor von vornherein zu vermeiden, sieht eine vorteilhafte Ausgestaltung der Erfindung vor, dass vor Zuführung des Filtratstromes in den Reaktor eine Trennung eines wässrigen Substrates in einen Konzentratstrom und in den Filterstrom erfolgt.

Damit es zu der für das Animpfkonzentrat erforderlichen Erzeugung und Vermehrung von Mikroorganismen kommt, sieht eine praktikable Variante der Erfindung vor, dass der Filtratstrom über die Füllkörper wandert. Hierdurch wird vorteilhafterweise erreicht, dass sich Mikroorganismen an dem Füllkörper immobilisieren und sich vermehren können.

Als ein probater Druckbereich, in dem der Reaktor effektiv arbeitet, hat sich ein Bereich zwischen 0 und 4 bar erwiesen.

Zudem sieht die Erfindung eine Biogasanlage zur Durchführung des Verfahrens vor, die zumindest eine Filtratleitung und einen mit Füllkörpern bestückten Reaktor aufweist, wobei der Reaktor mit der Filtratleitung verbunden ist.

Im Folgenden wird die Erfindung anhand der Zeichnung näher erläutert. Es zeigt in schematischer Darstellung:
- Fig. 1: ein Verfahren gemäß der Erfindung,

Fig. 2a und 2b Ausführungsformen des erfindungsgemäßen Reaktors.

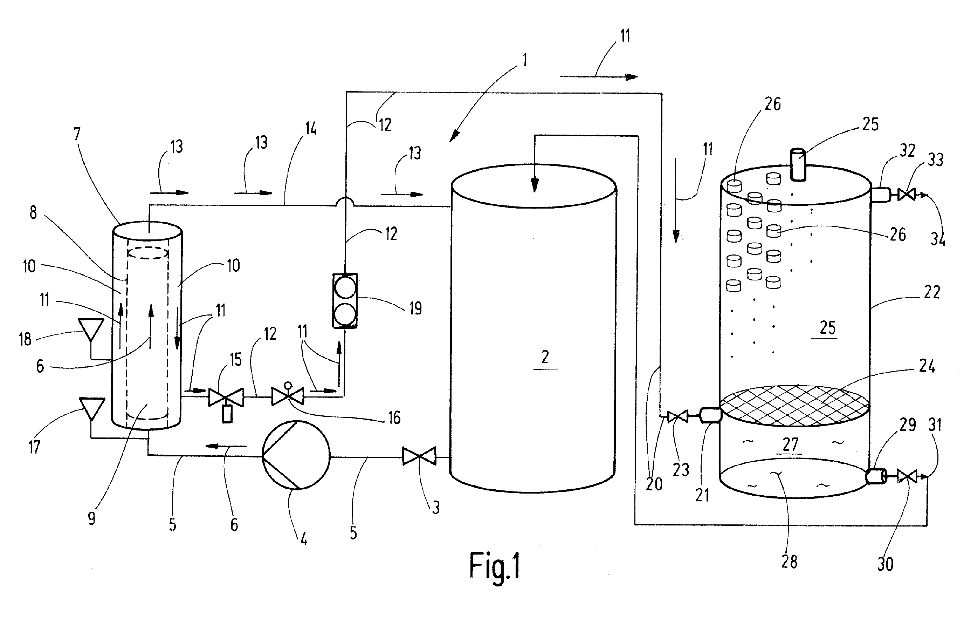

Das in Fig. 1 gezeigte erfindungsgemäße Verfahren beginnt in der Biogasanlage 1 mit einer Fest-Flüssig-Trennung, d.h. mit der Trennung des wässrigen Substrates aus dem Biogasreaktor 2 in einen Substrat- und Filterstrom.

Hierzu wird zunächst Substrat aus dem Bodenbereich des Biogasreaktors 2, in dem die Fermentation der Rohstoffe des Substrates erfolgt, mittels der Pumpe 4 über das Ventil 3 in die Substratleitung 5 gepumpt und durch die Substratleitung 5 als Substratstrom 6 in den als Cross-Flow-Filter ausgebildeten Filter 7 geführt. In dem Filter 7 erfolgt die Trennung des Substrates in ein energiereiches Konzentrat, welches einen hohen Feststoffgehalt und einen geringen Wassergehalt aufweist und in ein energiearmes Filtrat, welches sich durch einen geringen Feststoffgehalt und einen hohen Wasseranteil auszeichnet.

Für die Trennung in Filtrat und Konzentrat ist in dem Filter 7 ein Filtermedium 8 vorgesehen, das den Filter 7 in eine Substratseite 9, d.h. in einen Bereich des Filters 7 vor der Filterung, und in eine Filtratseite 10, d.h. in einen Bereich des Filters 7 nach der Filterung, unterteilt. Das Filtrat befindet sich somit auf der Filtratseite 10 und wird als Filtratstrom 11 in die Filtratleitung 12 transportiert. Das Konzentrat dagegen befindet sich auf der Substratseite 9 und wird als Konzentratstrom 13 in die Konzentratleitung 14 geleitet und weiter zum Biogasreaktor 2 transportiert, indem es zur weiteren Fermentation und Biogasproduktion verwendet werden kann.

In dem Filter 7 konkurrieren zwei für die Filtration relevanten Kräfte. Zum einen die horizontale Reibungskraft, die sich in dem Substratstrom 6 manifestiert und andererseits der Filtratstrom 11, der als vertikale Sogkraft wirkt. Um eine Verstopfung des Filters 7 zu vermeiden, müssen diese beiden Kräfte im Gleichgewicht sein. Partikel und Fasern, welche durch die Flusskraft des Filtrats 7 in Richtung des Filtermediums 8 gezogen werden, müssen von der Fluss- und Reibungskraft hierzu von dem Substratstrom 6 entfernt werden. Um die beiden relevanten Kräfte in dem Filter 7, d.h. die Reibungs- und Sogkraft, miteinander ins Gleichgewicht zu bringen, erfolgt eine Regelung des Filtratstromes 11 über den Druck.

Hierzu ist in der Filtratleitung 12 ein Absperrventil 15 und ein Regelventil 16 angeordnet. Mit dem Regelventil 16 erfolgt eine variable Regelung des Filtratstromes 11, wohingegen das Absperrventil 15 immer dann eingesetzt und geschlossen wird, wenn eine schnelle Reaktion des Regelventiles 16 ausbleibt.

Die Druckdifferenz zwischen Substratstrom 6 und Filtratstrom 11 fungiert als Regelgröße. Die Drücke des Substrat- 6 und Filtratstromes 11 werden dabei über die Drucksensoren 17, 18 gemessen. Ist der Druck auf der Substratseite 9 größer als auf der Filtratseite 10, so ist es das Bestreben der Flüssigkeit, wässrigen Substratstrom 6 in die Filtratseite 10 hineinzufiltrieren. Die Folge ist, dass hierdurch der Filtratstrom 11 steigt. Hierdurch verschiebt sich wiederum das Kräftegleichgewicht zwischen der horizontalen Reibungskraft und der vertikalen Sogkraft und der Filter 7 verstopft. Zur Schließung des Absperrventiles 15 wird nun der Filtratstrom 11 gänzlich unterdrückt, so dass ausschließlich der Substratstrom 6 als raue Komponente der durch die Strömung evtl. im Filter 7 befindliche Partikel und Fasern zusätzlich mit sich trägt und in dem Filter 7 wirkt, was mit einer selbstständigen Reinigung des Filters 7 einhergeht. Gleichzeitig zum Absperrventil 15 schließt auch das Regelventil 16, so dass nach Öffnung des Absperrventiles 15 der Filtratstrom 6 minimiert wird. Hierdurch wird verhindert, dass der Druck auf der Filtratseite 10 nach Öffnung des Absperrventiles 15 übermäßig sinkt.

Das Regelventil 16 wird so weit geöffnet, bis die Drücke auf der Substratseite 9, d.h. in dem Substratstrom 6 und auf der Filtratseite 10, d.h. in dem Filtratstrom 11, gleich sind. Die Folge hiervon ist, dass die Kraft zwischen Substratstrom 6 und Filtratstrom 11 gleich ist und der Trennungsprozess, d.h. die Trennung in Konzentrat und Filtrat wieder solange vonstatten geht bis es zu einem Rückstoß auf der Substratseite 9 bzw. einem Druckabfall auf der Filtratseite 10 kommt. Das Filtrat wird als Filtratstrom 11, wie bereits erwähnt durch die Filtratleitung 12 geführt und das Konzentrat als Konzentratstrom 13 über die Konzentratleitung 14 dem Biogasreaktor 2 zugeführt.

Zur Quantifizierung des Filtratstroms 11 ist in der Filtratleitung 12, und zwar den Ventilen 15, 16 nachgeschaltet, ein Durchflussmesser 19 in Gestalt eines magnetisch induktiven Durchflussmessers zur Messung des Filtratstromes 11 angeordnet. Die ermittelte Messgröße dient dabei als weitere Regelgröße zur Öffnung des Regelventiles 16. Das Regelventil 16 kann auch die Funktion erfüllen, sofern ein Kräftegleichgewicht in dem Filter 7 besteht, eine fest definierte Trennungsrate in dem Filter 7 einzustellen. Die Regelung der Ventile 15, 16 erfolgt durch herkömmliche Messelektronik.

In erfindungswesentlicher Weise wird der Filtratstrom 11 zur weiteren Verwendung über die Zulaufleitung 20 und den Zulaufstutzen 21 in den Reaktor 22 geleitet, wobei der Filtratstrom 11 durch das Ventil 23 unterbrochen werden kann.

Der Reaktor 22 ist zylinderförmig und weist einen Radius zwischen 50 und 300 mm und einer Höhe zwischen 300 und 3000 mm auf. Der Druck in dem Reaktor 22 liegt zwischen 0 und 4 bar.

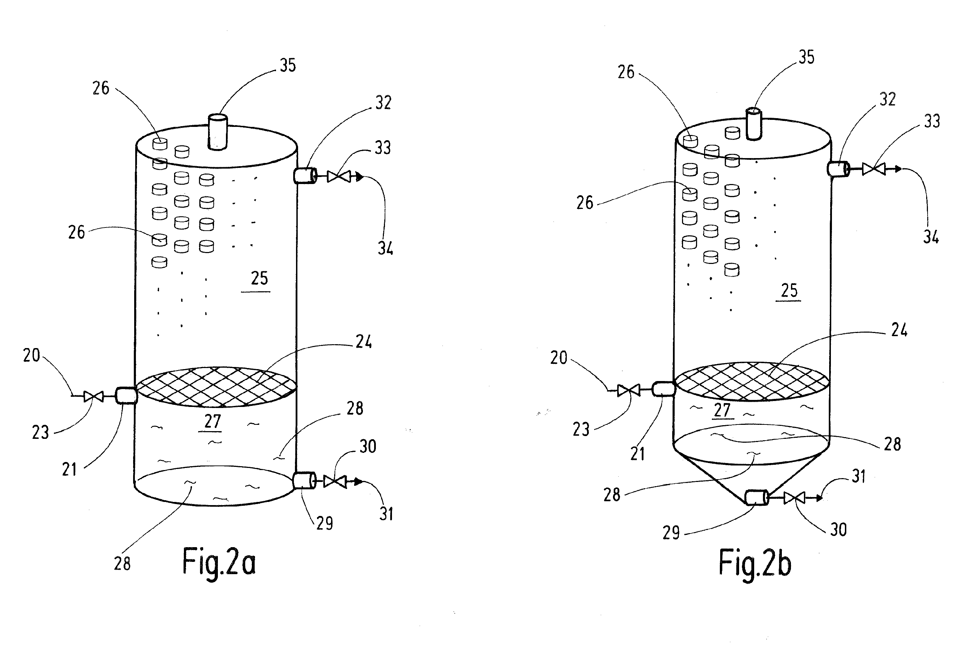

Der Reaktor 22 ist durch ein Auffanggitter 24 in zwei Bereiche unterteilt: einen oberen Teil 25, welcher mit hohlen zylindrischen Füllkörpern 26, analog zu einem Festbettreaktor beschickt ist, wobei in den Fig. 1 und 2a, 2b aus zeichnerischen Gründen nur eine begrenzte Anzahl der Füllkörper 26 dargestellt ist, und einem unteren Teil 27, welcher dazu dient, Sinkstoffe aufzunehmen. Hier sammelt sich das hochkonzentrierte Animpfkonzentrat 28.

Zur Entnahme des hochkonzentrierten Animpfkonzentrates 28 dient der Auslassstutzen 29, welcher über das Auslassventil 30 mit der Auslassleitung 31 verbunden ist.

Der über den Zulaufstutzen 21 eingebrachte Filtratstrom 11 wandert über die Füllkörper 26 - an dem Mikroorganismen immobilisieren und sich vermehren können - und wird über den Auslassstutzen 32, welcher über das Auslassventil 33 mit der Auslassleitung 34 verbunden ist, aus dem Reaktor 22 geführt. Um einen Überdruck zu verhindern, verfügt der Reaktor 22 über einen Überdruckstutzen 35, an dem eine handelsübliche Überdrucksicherung angeschlossen werden kann.

Die vorliegende Erfindung beschränkt sich in ihrer Ausführung nicht auf das vorstehend angegebene bevorzugte Ausführungsbeispiel. Vielmehr ist eine Anzahl von Varianten möglich, welche von der dargestellten Lösung auch bei grundsätzlich anders gearteten Ausführungen Gebrauch machen. So kann der Reaktor 22 unterschiedliche konstruktive Ausgestaltungen aufweisen. Er kann, wie aus Fig. 2a hervorgeht, die Ausgestaltung aufweisen, die der in Fig. 1 gezeigte und in die Biogasanlage 1 integrierte Reaktor 22 hat. Wie Fig. 2b zeigt, kann der Reaktor 22 aber auch im Bodenbereich nach unten konisch zulaufen, wobei am unteren Ende des Reaktors 22 der Auslassstutzen 29, das Auslassventil 30 und die Auslassleitung 31 angeordnet sind.

### Bezugszeichenliste:

- 1: Biogasanlage
- 2: Biogasreaktor
- 3, 23: Ventil
- 4: Pumpe
- 5: Substratleitung
- 6: Substratstrom
- 7: Filter
- 8: Filtermedium
- 9: Substratseite
- 10: Filtratseite
- 11: Filtratstrom
- 12: Filtratleitung
- 13: Konzentratstrom
- 14: Konzentratleitung
- 15: Absperrventil
- 16: Regelventil
- 17, 18: Drucksensoren
- 19: Durchflussmesser
- 20: Zulaufleitung
- 21: Zulaufstutzen
- 22: Reaktor
- 24: Auffanggitter
- 25: oberer Teil
- 26: Füllkörper
- 27: unterer Teil
- 28: Animpfkonzentrat
- 29, 32: Auslassstutzen
- 30, 33: Auslassventil
- 31, 34: Auslassleitung
- 35: Überdruckstutzen

## Patentansprüche

1. Verfahren zum Anfahren von Biogasanlagen (1), bei dem ein Animpfkonzentrat (28) erzeugt wird, mit folgenden Verfahrensschritten:
a) Erzeugung eines Filtratstromes (11) aus einem Filtrat;
b) Zuführung des Filtratstromes (11) in einen mit Füllkörpern (26) beschickten Reaktor (22);
c) Erzeugung des Animpfkonzentrates (28) in dem Reaktor (22);
d) Entnahme des Animpfkonzentrates (28) aus dem Reaktor (22);
e) Abführung des Filtratstromes (11) aus dem Reaktor (22).

2. Verfahren nach Anspruch 1,
**dadurch gekennzeichnet,**
**dass** vor Zuführung des Filtratstromes (11) in den Reaktor (22) eine Trennung eines wässrigen Substrates in einen Konzentratstrom (13) und in den Filterstrom (11) erfolgt.

3. Verfahren nach Anspruch 1 oder 2,
**dadurch gekennzeichnet,**
**dass** der Filtratstrom (11) über die Füllkörper (26) wandert.

4. Verfahren nach einem der Ansprüche 1 bis 3,
**dadurch gekennzeichnet,**
**dass** der Reaktor (22) bei einem Druck zwischen 0 und 4 bar arbeitet.

5. Biogasanlage (1) zur Durchführung des Verfahrens nach einem der vorangehenden Ansprüche mit zumindest einer Filtratleitung (12),
**dadurch gekennzeichnet,**
**dass** sie einen mit Füllkörpern (26) bestückten Reaktor (22) aufweist, der mit der Filtratleitung (12) verbunden ist.

6. Anlage nach Anspruch 5,
**dadurch gekennzeichnet,**
**dass** der Reaktor (22) im Inneren zweigeteilt ist, wobei der obere Teil (25) mit den Füllkörpern (26) beschickt ist und von einem unteren Teil (27) getrennt ist.

7. Anlage nach Anspruch 6,
**dadurch gekennzeichnet,**
**dass** zwischen oberem Teil (25) und dem unteren Teil (27) ein Gitter (24) angeordnet ist.

8. Anlage nach einem der Ansprüche 5 bis 7,
**dadurch gekennzeichnet,**
**dass** der Druck in dem Reaktor (22) zwischen 0 und 4 bar beträgt.

9. Anlage nach einem der Ansprüche 5 bis 8,
**dadurch gekennzeichnet,**
**dass** der obere Teil (25) einen Auslassstutzen (32) aufweist.

10. Anlage nach einem der Ansprüche 5 bis 9,
**dadurch gekennzeichnet,**
**dass** der untere Teil (27) über einen Auslassstutzen (29) verfügt.

11. Anlage nach Anspruch 9,
**dadurch gekennzeichnet,**
**dass** der Auslassstutzen (32) des oberen Teiles (25) mit einer Auslassleitung (34) verbunden ist.

12. Anlage nach Anspruch 10,
**dadurch gekennzeichnet,**
**dass** der Auslassstutzen (29) des unteren Teiles (27) mit einer Auslassleitung (34) verbunden ist.
